# EUROPÄISCHE PATENTSCHRIFT

(11) **EP 2 981 241 B1**
(45) Veröffentlichungstag und Bekanntmachung des Hinweises auf die Patenterteilung: **28.10.2020**
(21) Anmeldenummer: 14716538.5
(22) Anmeldetag: 01.04.2014
(51) Int. Cl.: A61M 5/162, A61J 1/20, A61M 39/26, A61M 39/10

(54) **FLUIDVENTIL- UND -VERBINDUNGSVORRICHTUNG**
FLUID VALVE AND FLUID CONNECTION SYSTEM
DISPOSITIF DE SOUPAPE ET DE LIAISON FLUIDIQUE

(30) Priorität: 02.04.2013 DE 102013205813
(43) Veröffentlichungstag der Anmeldung: 10.02.2016
(73) Patentinhaber: Parker Hannifin Manufacturing Germany GmbH & Co. KG, 33659 Bielefeld (DE)
(72) Erfinder: AYDIN, Tolga, 74206 Bad Wimpfen (DE)
(74) Vertreter: Grauel, Andreas
(86) Internationale Anmeldenummer: PCT/EP2014/056512
(87) Internationale Veröffentlichungsnummer: WO 2014/161846

(56) Entgegenhaltungen:
- EP-A1- 1 454 650
- WO-A1-93/20772
- WO-A1-96/17646
- WO-A1-98/14163
- WO-A1-2008/022040
- DE-A1-102010 047 747
- FR-A1- 2 952 813
- US-A- 5 280 876
- US-A- 5 390 898

## Beschreibung

### Technisches Gebiet

Die Erfindung betrifft eine Fluidventil- und -verbindungsvorrichtung, insbesondere zum Verbinden von Gefäßen und zur Erzeugung einer gesteuerten Fluidverbindung zwischen an der Fluidventil- und -verbindungsvorrichtung angeschlossenen Gefäßen bzw. Elementen.

### Stand der Technik

Insbesondere im medizinischen Anwendungsbereich ist es im Stand der Technik bekannt, dass flüssige Mittel bzw. Medikamente mittels Spritzen aus einem Vorratsgefäß aufgenommen werden und mit anderen Flüssigkeiten gemischt oder direkt Patienten verabreicht werden Derartige Fluidventilvorrichtungen sind zum Beispiel aus den Dokumenten US 5 280 876, US 5 390 898, WO 2008/022040, FR 2 952 813, WO 98/14163, EP 1 454 650, WO 96/17646, WO 93/20772 und DE 10 2010 047747 bekannt.

Auch ist es bekannt, dass verschiedene Mittel, wie beispielsweise Flüssigkeiten und/oder Feststoffe beispielsweise als Pulver o.ä., aus zwei verschiedenen Vorratsgefäßen miteinander gemischt werden, in dem das eine Mittel aus einem ersten Vorratsgefäß in ein zweites Vorratsgefäß überführt wird, in welchem das zweite Mittel vorrätig ist und die Mischung der Mittel dann in dem zweiten Vorratsgefäß erfolgt. Dazu ist beispielsweise eines der Mittel eine Flüssigkeit, die von dem ersten Vorratsgefäß in das zweite Vorratsgefäß überführt werden soll. So kann mittels eines Verbindungselements eine Fluidverbindung zwischen den beiden Vorratsgefäßen erzeugt werden. Dabei erfolgt der Übergang der Flüssigkeit aber nur mäßig bis gar nicht.

Wird hingegen in dem ersten Vorratsgefäß ein Unterdruck erzeugt, so wird nach Erzeugung der Fluidverbindung zwischen den beiden Vorratsgefäßen die Flüssigkeit aus dem ersten Vorratsgefäß in das zweite Vorratsgefäß eingesaugt. Ist das zweite Mittel in dem zweiten Vorratsgefäß ein Pulver, das es mit der Flüssigkeit zu vermischen gilt, so stellt das schlagartige Einsaugen der Flüssigkeit ein Problem dar, weil es unter gewissen Umständen auch zu ungleichmäßiger Mischung oder Klumpenbildung führen kann, was die Mischung unbrauchbar machen kann. Bei teuren Medikamenten gilt es dies möglichst zu vermeiden.

### Darstellung der Erfindung, Aufgabe, Lösung, Vorteile

Es ist die Aufgabe der Erfindung, eine Fluidventil- und -verbindungsvorrichtung zu schaffen, welche eine sichere Fluidverbindung erlaubt und dennoch auch einen kontrollierten Fluidstrom zwischen zwei Gefäßen zulässt.

Dies wird erreicht mit den Merkmalen von Anspruch 1.

Ein vorteilhaftes Ausführungsbeispiel der Erfindung betrifft eine Fluidventil- und -verbindungsvorrichtung mit einem Gehäuse mit einem Aufnahmeraum mit einem ersten internen Anschlusselement und mit einem ersten externen Anschlusselement, mit einem in dem Aufnahmeraum angeordneten ersten verlagerbaren Ventilelement, und mit einem Betätigungselement, welches eine Fluidkommunikationsvorrichtung mit einem zweiten internen Anschlusselement und mit einem zweiten externen Anschlusselement aufweist, wobei das erste interne Anschlusselement mit dem zweiten internen Anschlusselement und dem Ventilelement zusammen wirken, um eine Fluidverbindung zwischen dem ersten und dem zweiten externen Anschlusselement zu bewirken oder zu unterbinden. Dabei kann durch die gesteuert schaltbare Fluidverbindung eine gezielte Zusammenführung zweier Medien, wie Mittel vorgenommen werden, so dass eine gezielte Vermischung stattfinden kann.

Dabei ist das Betätigungselement relativ zu dem Gehäuse verlagerbar und nimmt eine erste Position ein, in welcher die Fluidverbindung zwischen dem ersten und dem zweiten externen Anschlusselement unterbunden ist und eine zweite Position, in welcher die Fluidverbindung zwischen dem ersten und dem zweiten externen Anschlusselement bewirkt ist. Durch die gezielte Verlagerung des Betätigungselements von einer ersten in eine zweite Position erfolgt die Erzeugung einer Fluidverbindung durch Verlagerung eines Elements der Vorrichtung. Wird diese Verlagerung gezielt verhindert, so kann auch das Erzeugen der Fluidverbindung verhindert werden bzw. bei Erlauben der Fluidverbindung gezielt erzeugt werden.

Das Betätigungselement weist Mittel auf, welche mit Mitteln des Gehäuses zusammenwirken, um die erste und die zweite Position zu definieren. Dies ist vorteilhaft, wenn es gilt eine vordefinierte Position einzunehmen oder das Einnehmen dieser Position zu verhindern.

Dabei weisen die Mittel des Betätigungselements zumindest einen Arm auf, welcher zumindest einen nach radial innen ausgerichteten Vorsprung aufweist, und die Mittel des Gehäuses weisen in einer Gehäusewandung eingebrachte Nuten auf. Die Nuten sind dabei bevorzugt in radialer Richtung ausgerichtet, damit der zumindest eine Vorsprung in eine der Nuten eingreifen kann. Durch ein Verschieben des Betätigungselements in axialer Richtung kann von der ersten Position zur zweiten Position das Eingreifen von einer Nut in die andere Nut erfolgen. Damit wird eine definierte Verschiebung sichergestellt.

Dabei ist es zweckmäßig, wenn die Nuten in axialer Richtung des Gehäuses beabstandet zueinander angeordnet sind. So wird der Abstand zwischen den beiden Positionen definiert.

Auch ist es zweckmäßig, wenn der zumindest eine Arm in der ersten Position derart angeordnet ist, dass der zumindest eine Vorsprung in einer der Nuten eingreift und der zumindest eine Arm in der zweiten Position derart angeordnet ist, dass der zumindest eine Vorsprung in einer andern der Nuten eingreift. So wird die Verlagerung von der ersten zur zweiten Position im Abstand definiert.

Besonders vorteilhaft ist es, wenn ein Blockadeelement vorgesehen ist, welches bei Vorliegen in einer ersten Lage das Einnehmen der zweiten Position des Betätigungselements verhindert und welches bei Vorliegen in einer zweiten Lage das Einnehmen der zweiten Position des Betätigungselements erlaubt. Das Blockadeelement wird dabei als Verhinderer einer Fluidverbindung eingesetzt. Dies erlaubt es, die erfindungsgemäße Vorrichtung an beiden externen Anschlusselementen an ein Gefäß, wie Vorratsgefäß anzuschließen, ohne dass dadurch eine Fluidverbindung erzeugt wird. Erst durch Verlagerung oder Entfernung des Blockadeelements wird eine Verlagerung des Betätigungselements erlaubt, was die Fluidverbindung erzeugt.

Besonders vorteilhaft ist es, wenn das Blockadeelement ein offenes Ringelement ist, welches um das Gehäuse greift und das Einnehmen der zweiten Position des Betätigungselements dadurch verhindert, dass die Lage der Mittel des Betätigungselements derart begrenzt wird, dass der zumindest eine Vorsprung nicht in die der zweiten Position zugeordneten Nut eingreifen kann. Durch die Gestaltung kann das Blockadeelement günstig eingeführt werden, so dass es das Betätigungselement auf eine Position fixiert bzw. die zweite Position nicht einnehmen lässt.

Dabei ist es zweckmäßig, wenn das Gehäuse mit Befestigungsmitteln versehen ist, um das Gehäuse an einem zweiten Vorratsgefäß zu befestigen. So kann das Gehäuse fest an das Vorratsgefäß angeschlossen werden, damit keine unbeabsichtigten Leckagen bzw. Mittelverluste auftreten.

Auch ist es zweckmäßig, wenn das Gehäuse mit dem zweiten externen Anschlusselement versehen ist, um eine Fluidverbindung mit dem zweiten Vorratsgefäß zu bewirken.

Weiterhin ist es zweckmäßig, wenn das Betätigungselement mit Befestigungsmitteln versehen ist, um das Gehäuse an einem ersten Vorratsgefäß zu befestigen.

Auch ist es vorteilhaft, wenn das Betätigungselement mit dem ersten externen Anschlusselement versehen ist, um eine Fluidverbindung mit dem ersten Vorratsgefäß zu bewirken.

Dabei ist es vorteilhaft, wenn die Befestigungsmittel nach Art von Rastarmen ausgebildet sind. Dadurch lässt sich eine werkzeuglose Verbindung erzielen, die auch leicht wieder manuell lösbar ist.

Ebenso ist es zweckmäßig, wenn das erste und/oder das zweite Anschlusselement nach Art einer Hohlkanüle oder als Hohldorn ausgebildet ist. Dadurch kann das Vorratsgefäß mit einem Deckel durchstoßen werden, um die Fluidverbindung zu erzeugen.

Besonders vorteilhaft ist es, wenn das erste interne Anschlusselement ein hohlzylindrischer Stutzen ist und das zweite interne Anschlusselement ein hohlzylindrischer Stutzen ist, welche bei Verlagerung zwischen der ersten Position und der zweiten Position ineinander greifen. Dadurch kann eine sichere Fluidverbindung erzeugt werden.

Auch ist es zweckmäßig, wenn das zweite interne Anschlusselement ein hohlzylindrischer Stutzen ist, welcher bei Verlagerung zwischen der ersten Position und der zweiten Position das Ventilelement beaufschlagt. Dadurch wird das Ventilelement bei Betätigung des Betätigungselements automatisch betätigt.

Auch ist es zweckmäßig, wenn das Ventilelement in der ersten Position des Betätigungselements die Fluidverbindung zwischen dem ersten und dem zweiten externen Anschlusselement unterbindet und das Ventilelement in der zweiten Position des Betätigungselements die Fluidverbindung zwischen dem ersten und dem zweiten externen Anschlusselement ermöglicht.

Besonders vorteilhaft ist es, wenn das Ventilelement einen etwa zylindrischen Bereich mit von dem zylindrischen Bereich abragende Federarme aufweist.

Dabei ist es auch vorteilhaft, wenn das Ventilelement mit seinem etwa zylindrischen Bereich in dem ersten internen Anschlusselement angeordnet ist, wobei sich die Federarme jeweils an einer Innenwandung des Gehäuses abstützen.

Weitere vorteilhafte Ausgestaltungen sind durch die nachfolgende Figurenbeschreibung und durch die Unteransprüche beschrieben.

### Kurze Beschreibung der Zeichnungen

Nachstehend wird die Erfindung auf der Grundlage zumindest eines Ausführungsbeispiels anhand der Zeichnungen näher erläutert. Es zeigen:
- Fig. 1: einen Schnitt durch ein Ausführungsbeispiel einer erfindungsgemäßen Fluidventil- und - verbindungsvorrichtung in einer ersten Betriebsstellung,
- Fig. 2: einen Schnitt durch ein Ausführungsbeispiel einer erfindungsgemäßen Fluidventil- und - verbindungsvorrichtung in einer zweiten Betriebsstellung,
- Fig. 3: eine Seitenansicht der erfindungsgemäßen Fluidventil- und -verbindungsvorrichtung in der ersten Betriebsstellung,
- Fig. 4: eine Explosionsdarstellung der erfindungsgemäßen Fluidventil- und -verbindungsvorrichtung,
- Fig. 5: einen Schnitt durch ein weiteres Ausführungsbeispiel einer erfindungsgemäßen Fluidventil- und - verbindungsvorrichtung, und
- Fig. 6: eine Ansicht von oben gemäß Fig. 5.

### Bevorzugte Ausführung der Erfindung

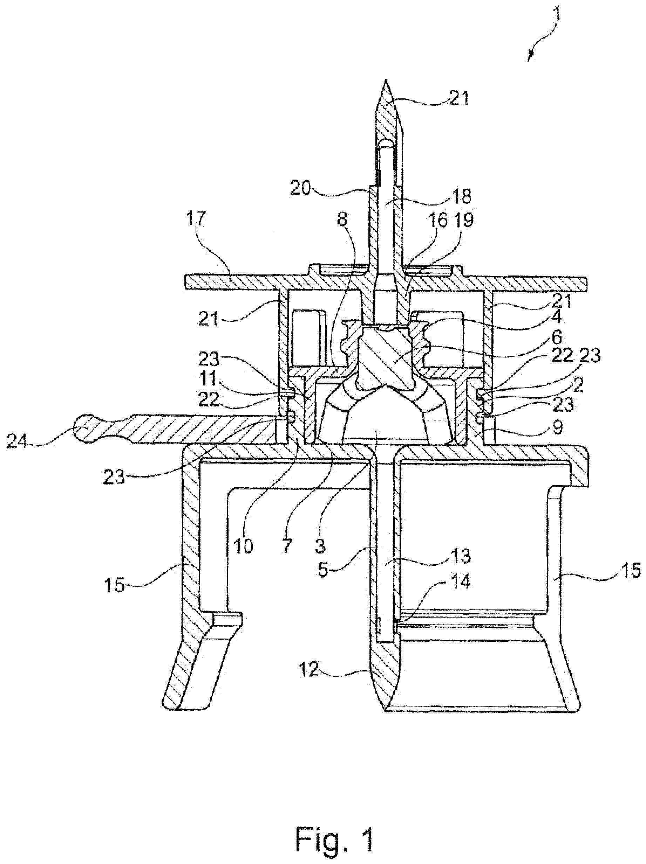

Die Figur 1 zeigt eine Fluidventil- und -verbindungsvorrichtung 1 mit einem Gehäuse 2 mit einem in dem Gehäuse 2 angeordneten Aufnahmeraum 3 mit einem ersten internen Anschlusselement 4 und mit einem ersten externen Anschlusselement 5. Dabei kann eine Fluidverbindung zwischen dem ersten internen Anschlusselement 4 und dem ersten externen Anschlusselement 5 erzeugt bzw. unterbrochen werden, in dem ein Ventilelement 6 so in dem Aufnahmeraum 3 bzw. in dem ersten internen Anschlusselement 4 angeordnet ist, um eine Fluidverbindung zu erlauben beziehungsweise zu blockieren.

Das Gehäuse 2 besteht aus einer bodenseitigen Wandung 7, einer dieser gegenüberliegenden deckelseitigen Wandung 8 sowie einer im Wesentlichen ringzylindrischen Wandung 9, welche die bodenseitige Wandung 7 mit der deckelseitigen Wandung 8 verbindet.

Im Ausführungsbeispiel der Figur 1 ist die ringförmige Wandung 9 doppelwandig ausgebildet, wobei eine Wandung 10 von der bodenseitigen Wandung 7 in axialer Richtung abragt und eine Wandung 11 von der deckelseitigen Wandung 8 in axialer Richtung derart abragt, dass die beiden Wandungen 10, 11 radial ineinander angeordnet sind und miteinander verbindbar sind. So kann die Wandung 10 beispielsweise mit der Wandung 11 verbunden werden, wie bevorzugt verschweißt oder verklebt werden.

Bei einem weiteren Ausführungsbespiel kann auch eine der Wandungen 10, 11 eingespart sein, so dass die verbleibende eine Wandung 10, 11 dann entweder mit der bodenseitigen Wandung 7 oder der deckelseitigen Wandung 8 einteilig ausgebildet ist und mit der jeweils anderen Wandung 8, 7 verbunden ist.

Bei einem weiteren Ausführungsbeispiel kann die Wandung 9 auch als separates Element ausgebildet sein und sowohl mit der bodenseitigen Wandung 7 als auch mit der deckelseitigen Wandung 8 verbindbar sein.

Das erste interne Anschlusselement 4 ist bevorzugt ein hohlzylindrischer Anschlussstutzen, welcher von der deckelseitigen Wandung 8 in axialer Richtung abragt. Das erste externe Anschlusselement 5 ist bevorzugt eine Hohlkanüle oder ein Hohldorn, welcher in axialer Richtung von der bodenseitigen Wandung 7 abragt. Dabei ragt das erste externe Anschlusselement 5 bevorzugt in entgegengesetzter Richtung von der Wandung 7 ab, als das erste interne Anschlusselement 4 von der deckelseitigen Wandung 8 abragt.

Das erste externe Anschlusselement 5 ist, wie in Figur 1 zu erkennen, als Hohlkanüle oder als Hohldorn ausgebildet, wobei der Dorn beziehungsweise die Kanüle an seinem vorderen Endbereich 12 spitz zulaufend ausgebildet ist, um durch einen Deckel eines Vorratsgefäßes gestoßen werden zu können. In dem Hohldorn beziehungsweise in der Hohlkanüle ist ein Fluidpfad 13 vorgesehen, welcher eine Fluidverbindung zwischen dem Aufnahmeraum 3 und einem Vorratsgefäß bewirken kann, wobei die Austrittsöffnung 14 des ersten externen Anschlusselements 5 als Hohldorn bzw. als Hohlkanüle bevorzugt an einer Seitenwandung der Hohlkanüle bzw. des Hohldorns angeordnet ist. Dies bewirkt, dass wenn ein Fluid aus dem Aufnahmeraum 3 durch den Fluidpfad 13 und die Austrittsöffnung 14 strömt, der Fluidstrom in radialer Richtung austritt und bevorzugt gegen eine Seitenwandung des Vorratsgefäßes strömt. Von dort kann das Fluid oder Mittel beispielsweise an der Wandung entlang zum Boden des Vorratsgefäßes strömen. Dies sorgt für eine verbesserte Vermischung der zu mischenden Fluide bzw. Mittel.

Die bodenseitige Wandung 7 weist an ihren radialen Endbereichen Befestigungsmittel 15 auf, die nach Art von Rasthaken ausgebildet sind, um ein Vorratsgefäß sicher mit der Fluidventil- und -verbindungsvorrichtung verbinden zu können.

Zur Verbindung eines Vorratsgefäßes mit der erfindungsgemäßen Vorrichtung mittels der Befestigungselemente 15 kann ein Vorratsgefäß von unten in Figur 1 kommend auf den Dorn des ersten externen Anschlusselements 5 aufgeschoben werden, so dass das Gefäß gleichzeitig mittels der als Rasthaken ausgebildeten Befestigungselemente gesichert wird.

Weiterhin umfasst die erfindungsgemäße Vorrichtung 1 ein Betätigungselement 16, welches eine Grundplatte 17 aufweist, weiche einen Fluidkanal 18 trägt, der auf einer Seite der Grundplatte 17 durch das zweite interne Anschlusselement 19 gebildet wird und auf der anderen Seite der Grundplatte durch das zweite externe Anschlusselement 20.

Das zweite interne Anschlusselement 19 wird durch einen Rohrstutzen gebildet, der im Wesentlichen koaxial zu dem ersten internen Anschlusselement 4 ausgebildet und angeordnet ist. Das zweite externe Anschlusselement 20 ist bevorzugt als Hohldorn gebildet, der an der vorderen Seite des Hohldorns eine Öffnung 21 aufweist, um bei einem Durchstoßen eines Deckels eines Vorratsgefäßes eine Fluidverbindung zwischen dem zweiten externen Anschlusselement 20 und dem zweiten internen Anschlusselement 19 zu erlauben.

Weiterhin weist das Betätigungselement 16 Mittel auf, die mit Mitteln des Gehäuses zusammen wirken, um die Positionierung des Betätigungselements 16 relativ zu dem Gehäuse 2 zu definieren. Als betätigungselementseitige Mittel ist zumindest ein Arm oder eine Mehrzahl von Armen 21 vorgesehen, die sich in axialer Richtung erstrecken und die an ihren Endbereichen sich in radialer Richtung erstreckende Vorsprünge 22 aufweisen. Diese Vorsprünge 22 können in Nuten 23 der Wand 9 des Gehäuses eingreifen, die bevorzugt umlaufend in der Wand 9 eingebracht sind. Dabei sind die Nuten 23 derart beabstandet, dass bei Eingreifen der Vorsprünge 22 in die erste Nut 23, die oberste Nut 23, das Betätigungselement 16 in seiner ersten Position angeordnet ist und bei Eingreifen der Vorsprünge 22 in der zweiten, unteren Nut 23, das Betätigungselement 16 in seiner zweiten Position angeordnet ist.

Weiterhin ist ein Blockadeelement 24 vorgesehen, dass bei Einnahme seiner Sollposition lediglich erlaubt, dass das Betätigungselement 16 in seiner ersten Position angeordnet ist, weil die Einnahme der Vorsprünge 22 in die Nut 23 der zweiten Position blockiert wird.

In Figur 1 ist zu erkennen, dass die Vorsprünge 22 der Arme 21 in der oberen der beiden Nuten 23 angeordnet ist und damit das zweite interne Anschlusselement 19 nicht oder nur geringfügig in die Öffnung des ersten internen Anschlusselements 4 eingreift und das verlagerbare Ventilelement 6 nicht oder nur geringfügig beaufschlagt, so dass eine Unterbindung der Fluidverbindung zwischen dem ersten externen Anschlusselement 5 und dem zweiten externen Anschlusselement 20 vorliegt.

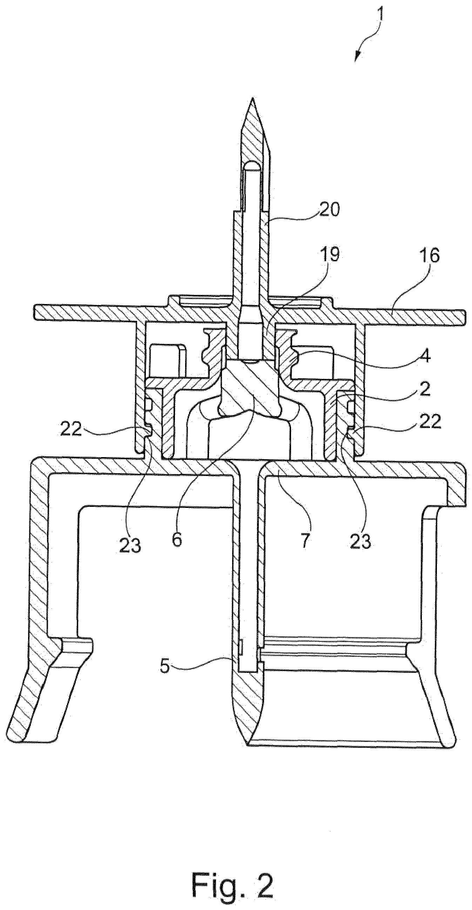

In Figur 2 ist das Betätigungselement 16 relativ zu dem Gehäuse 2 derart verlagert, dass die Vorsprünge 22 in die untere der beiden Nuten 23 eingreifen und das Betätigungselement relativ zu dem Gehäuse seine zweite Position einnimmt. In dieser zweiten Position greift das zweite interne Anschlusselement 19 in das erste interne Anschlusselement 4 ein und beaufschlagt das Ventilelement 6 derart, dass das Ventilelement 6 in Richtung auf die Wandung 7 verformt oder verlagert wird, um eine Fluidverbindung zwischen dem ersten externen Anschlusselement 5 und dem zweiten externen Anschlusselement 20 zu bewirken.

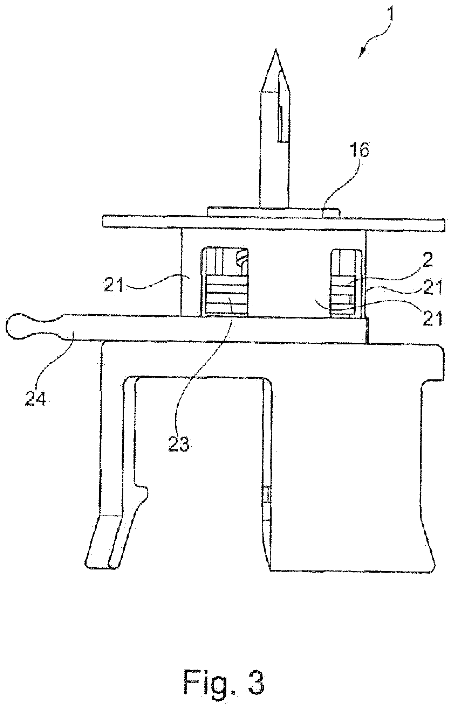

Die Figur 3 zeigt eine Ansicht der erfindungsgemäßen Fluidventil- und -verbindungsvorrichtung 1 mit dem Gehäuse 2 und dem Betätigungselement 16, wobei zu erkennen ist, dass die Arme 21 als kreissegmentartig gewölbte Arme ausgebildet sind, die an ihrer Innenwandung die Vorsprünge 22 aufweisen, welche in die Nuten 23 eingreifen können. Das Blockadeelement 24 ist als ringförmiges Element mit einem offenen Ring ausgebildet, so dass es in lateraler beziehungsweise radialer Richtung aus dem Verbund herausgezogen werden kann, um die Verlagerbarkeit des Betätigungselements 16 in axialer Richtung zwischen der ersten und der zweiten Position zu ermöglichen.

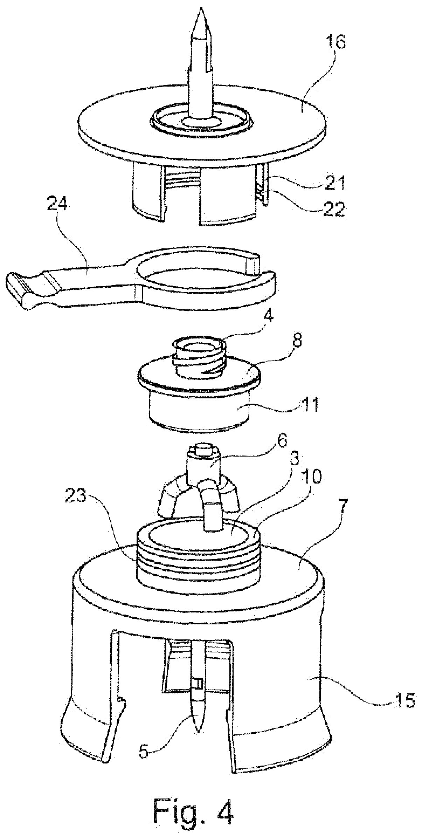

Die Figur 4 zeigt die erfindungsgemäße Vorrichtung in einer Explosionsdarstellung. Man erkennt, dass die bodenseitige Wandung 7 mit der Wandung 10, welche die Nuten 23 trägt, einstückig ausgebildet werden kann, wobei das verlagerbare Ventilelement 6 auch in den Aufnahmeraum 3 einfügbar ist.

Anschließend kann das Element mit der deckelseitigen Wandung und der Wand 11 und dem ersten internen Anschlusselement 4 in den Aufnahmeraum 3 angeordnet und abgedichtet verbunden werden. Auf die entsprechende Vorrichtung kann anschließend das Betätigungselement 16 aufgesetzt werden, so dass unter Zwischeneinfügung des Blockadeelements 24 das Betätigungselement lediglich die erste Position einnehmen kann, so dass die Vorsprünge 22 lediglich in die obere der beiden Nuten 23 eingreifen können, um das Betätigungselement 16 in der ersten Position zu fixieren. Dazu ist das Blockadeelement 24 als offener Ring mit Griffelementen ausgebildet, so dass der offene Ring über das Gehäuse 2 außen geschoben werden kann, um die axiale Position des Betätigungselements 16 relativ zu dem Gehäuse 2 zu begrenzen.

Das Ventilelement 6 weist einen zylindrischen Bereich auf, von dem nach unten Federarme abragen. Dabei ist der zylindrische Bereich mit den Federarmen vorteilhaft einteilig ausgebildet und die Federarme ragen in einem Winkel von etwa 30° bis 60°, wie vorzugsweise 45°, zur Vertikalen nach radial außen und unten von dem zylindrischen Bereich ab. Die Federarme können gerade ausgebildet sein oder sie können alternativ auch abgewinkelt sein.

Die geraden Federarme bilden bei Vorhandensein von drei Federarmen eine Anordnung gemäß einer Kantenanordnung eines Tetraeders. Für die abgewinkelten Federarme gilt etwa das Gleiche für die an den zylindrischen Bereich anschließenden Teile der Federarme.

Am oberen Ende des zylindrischen Bereichs des Federelements ist eine kreuzförmige oder sternförmige Nutstruktur eingebracht, welche dazu dient, eine Fluidverbindung zwischen dem ersten und dem zweiten externen Anschlusselement zu gewährleisten.

In den Figuren 1 und 2 gezeigten Positionen des Ventilelements 6 ist eine Fluidverbindung zwischen dem ersten und dem zweiten externen Anschlusselement vorgesehen oder abgeschlossen und abgedichtet. Wird der zylindrische Bereich des Ventilelements beaufschlagt und nach unten geschoben, so stützen sich die Federarme in den Ecken des Aufnahmeraums oder am Boden ab und werden elastisch verformt. Dies wird soweit erfolgen, bis eine Fluidverbindung vorliegt.

Wird das beaufschlagende Element wieder aus dem internen Anschlusselement 4 entfernt, so entspannt sich das elastische formbare Ventilelement 6 wieder und der zylindrische Bereich wird wieder nach oben geschoben, so dass das interne Anschlusselement 4 wieder abgedichtet ist.

Bevorzugt ist die Vorrichtung, also das Gehäuse, das Betätigungselement und/oder das Blockadeelement aus Polycarbonat ausgebildet. Ebenso ist es vorteilhaft, wenn das Ventilelement aus einem Gummi, wie beispielsweise aus LSR, Liquid Silicone Rubber, ausgebildet ist.

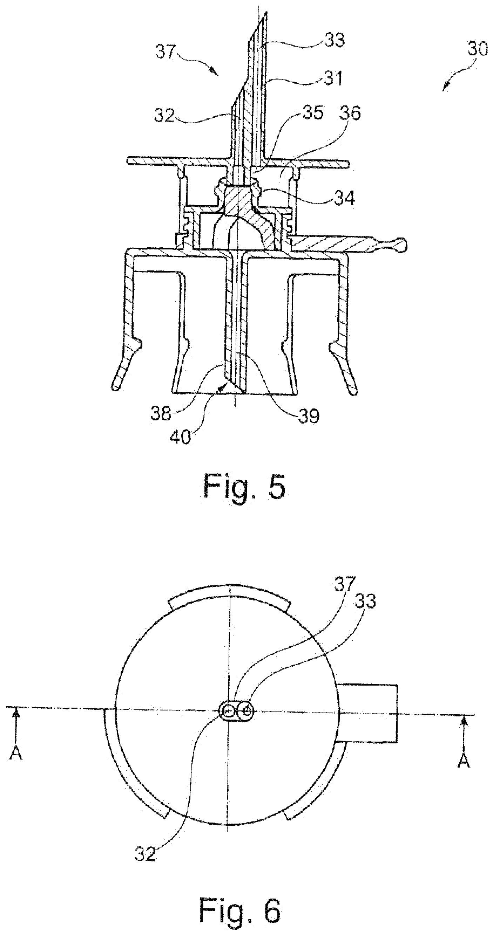

Die Figuren 5 und 6 zeigen ein weiteres Ausführungsbeispiel der Erfindung, bei welchem gegenüber dem in den Figuren 1 bis 4 gezeigten Ausführungsbeispiel die Anschlusselemente eine abgeänderte Gestalt aufweisen. Die anderen Merkmale sind im Wesentlichen unverändert und werden daher nicht weiter beschrieben. Diesbezüglich wird auf die Figurenbeschreibung der Figuren 1 bis 4 verwiesen.

In Figur 5 ist das zweite externe Anschlusselement 31 der Fluidventil- und -verbindungsvorrichtung 30 als Doppelkanal mit den Kanälen 32,33 ausgebildet. Dabei ist der Kanal 32 kürzer ausgebildet als der Kanal 33. Die oberen Endbereiche der Kanäle sind dabei optional abgeschrägt ausgebildet, wobei der obere Endbereich des Kanals 33 eine Spitze bildet, welche dazu geeignet ist, durch eine Verschlusskappe eines Gefäßes gestoßen zu werden. Auch der obere Endbereich des Kanals 32 ist diesbezüglich vorteilhaft abgeschrägt.

Der Kanal 32 führt an seinem unteren Endbereich in das erste interne Anschlusselement 34, wobei ein Stutzen 35 des Kanals 32 in das erste interne Anschlusselement 34 eingreift. Der Kanal 33 weist an seinem unteren Endbereich eine Öffnung auf, die neben dem Stutzen 35 liegt und welche in dem nach außen offenen Raum 36 mündet.

Diese Doppelkanalstruktur bewirkt für den Fall, dass in dem Gefäß, welches mit dem Dorn 37 verbunden ist, eine Flüssigkeit ist, und in dem Gefäß, welches mittels des Dorns 38 verbunden ist, ein Vakuum vorherrscht, dass die Flüssigkeit aus dem einen Gefäß in das zweite Gefäß gesaugt wird, wobei die Flüssigkeit durch den Kanal 32 strömt. Während dessen wird Luft durch den Kanal 33 in das erste Gefäß angesaugt, was den Fluidstrom vom ersten Gefäß zum zweiten Gefäß fördert, weil sich in dem ersten Gefäß kein Unterdruck bilden kann.

Die Figur 6 zeigt die beiden Kanäle 32, 33 in dem Dorn 37 von oben.

Der Dorn 38 als erstes externes Abschlusselement ist als nach unten offenes Rohr mit einem Kanal 39 mit einer endseitigen Abschrägung 40 ausgebildet. Mit dem Dorn 38 wird bevorzugt ein Gefäß fluidverbunden, das am unteren Bereich der Fluidventil- und -verbindungsvorrichtung 30 angeordnet wird.

Weiterhin ist es bevorzugt, wenn in dem ersten Kanal 32 und/oder in dem zweiten Kanal 33 ein Filter vorgesehen ist. Dieser Filter kann beispielsweise ein Papierfilter oder ähnliches sein, welcher in einem der Kanäle 32, 33 angeordnet oder vor- oder nachgeschaltet ist. So könnte beispielsweise ein Filter in dem Stutzen 35 angeordnet sein.

### Bezugszeichenliste

- 1: Fluidventil- und -verbindungsvorrichtung
- 2: Gehäuse
- 3: Aufnahmeraum
- 4: erstes internes Anschlusselement
- 5: erstes externes Anschlusselement
- 6: Ventilelement
- 7: Wandung
- 8: Wandung
- 9: Wandung
- 10: Wand
- 11: Wand
- 12: Endbereich
- 13: Fluidpfad
- 14: Austrittsöffnung
- 15: Befestigungselement
- 16: Betätigungselement
- 17: Grundplatte
- 18: Fluidkanal
- 19: zweites internes Anschlusselement
- 20: zweites externes Anschlusselement
- 21: Arm
- 22: Vorsprung
- 23: Nut
- 24: Blockadeelement

## Patentansprüche

1. Fluidventil- und -verbindungsvorrichtung (1) mit einem Gehäuse (2) mit einem Aufnahmeraum (3) mit einem ersten internen Anschlusselement (4) und mit einem ersten externen Anschlusselement (5), mit einem in dem Aufnahmeraum (3) angeordneten ersten verlagerbaren Ventilelement (6), und mit einem Betätigungselement (16), welches eine Fluidkommunikationsvorrichtung mit einem zweiten internen Anschlusselement (19) und mit einem zweiten externen Anschlusselement (20) aufweist, wobei das erste interne Anschlusselement (4) mit dem zweiten internen Anschlusselement (19) und dem Ventilelement (6) zusammen wirken, um eine Fluidverbindung zwischen dem ersten und dem zweiten externen Anschlusselement (5, 20) zu bewirken oder zu unterbinden, wobei der Aufnahmeraum (3) in dem Gehäuse (2) angeordnet ist,
das erste interne Anschlusselement (4) und das erste externe Anschlusselement (5) ragen von jeweils einer Wandung des Gehäuses (2) in axialer Richtung ab,
das erste interne Anschlusselement (4) und das erste externe Anschlusselement (5) sind über den Aufnahmeraum (3) miteinander verbindbar,
wobei durch das Verlagern des Betätigungselements (16) das Ventilelement (6) betätigt wird, um eine Fluidverbindung zwischen dem ersten externen Anschlusselement (5) und dem zweiten externen Anschlusselement (20) zu bewirken,
wobei das Betätigungselement (16) relativ zu dem Gehäuse (2) verlagerbar ist und eine erste Position einnehmen kann, in welcher die Fluidverbindung zwischen dem ersten und dem zweiten externen Anschlusselement (5, 20) unterbunden ist und eine zweite Position einnehmen kann, in welcher die Fluidverbindung zwischen dem ersten und dem zweiten externen Anschlusselement (5, 20) bewirkt ist,
wobei das Betätigungselement (16) Mittel (21, 22) aufweist, welche mit Mitteln (23) des Gehäuses (2) zusammenwirken, um die erste und die zweite Position zu definieren,
wobei die Mittel des Betätigungselements (16) zumindest einen Arm (21) aufweisen, welcher zumindest einen nach radial innen ausgerichteten Vorsprung (22) aufweist, und wobei die Mittel des Gehäuses (2) in einer Gehäusewandung eingebrachte Nuten (23) sind,
und ein Blockadeelement (24) vorgesehen ist, welches bei Vorliegen in einer ersten Lage das Einnehmen der zweiten Position des Betätigungselements (16) verhindert und welches bei Vorliegen in einer zweiten Lage das Einnehmen der zweiten Position des Betätigungselements (16) erlaubt.

2. Fluidventil- und -verbindungsvorrichtung (1) nach Anspruch 1, **dadurch gekennzeichnet, dass** die Nuten (23) in axialer Richtung des Gehäuses (2) beabstandet zueinander angeordnet sind.

3. Fluidventil- und -verbindungsvorrichtung (1) nach Anspruch 1 oder 2, **dadurch gekennzeichnet, dass** der zumindest eine Arm in der ersten Position derart angeordnet ist, dass der zumindest eine Vorsprung (22) in einer der Nuten (23) eingreift und der zumindest eine Arm (21) in der zweiten Position derart angeordnet ist, dass der zumindest eine Vorsprung (22) in einer anderen der Nuten (23) eingreift.

4. Fluidventil- und -verbindungsvorrichtung (1) nach einem der vorhergehenden Ansprüche, **dadurch gekennzeichnet, dass** das Blockadeelement (24) ein offenes Ringelement ist, welches um das Gehäuse (2) greift und das Einnehmen der zweiten Position des Betätigungselements (16) dadurch verhindert, dass die Lage der Mittel (23) des Betätigungselements (16) derart begrenzt wird, dass der zumindest eine Vorsprung (22) nicht in die der zweiten Position zugeordnete Nut (23) eingreifen kann.

5. Fluidventil- und -verbindungsvorrichtung (1) nach einem der vorhergehenden Ansprüche, **dadurch gekennzeichnet, dass** das Gehäuse (2) mit Befestigungsmitteln (15) versehen ist, um das Gehäuse (2) an einem zweiten Vorratsgefäß zu befestigen.

6. Fluidventil- und -verbindungsvorrichtung (1) nach einem der vorhergehenden Ansprüche, **dadurch gekennzeichnet, dass** das Gehäuse (2) mit dem ersten externen Anschlusselement (5) versehen ist, um eine Fluidverbindung mit dem zweiten Vorratsgefäß zu bewirken.

7. Fluidventil- und -verbindungsvorrichtung (1) nach einem der vorhergehenden Ansprüche, **dadurch gekennzeichnet, dass** das Betätigungselement (16) mit Befestigungsmitteln (15) versehen ist, um das Gehäuse (2) an einem ersten Vorratsgefäß zu befestigen.

8. Fluidventil- und -verbindungsvorrichtung (1) nach einem der vorhergehenden Ansprüche, **dadurch gekennzeichnet, dass** das Betätigungselement (16) mit dem zweiten externen Anschlusselement (20) versehen ist, um eine Fluidverbindung mit dem ersten Vorratsgefäß zu bewirken.

9. Fluidventil- und -verbindungsvorrichtung (1) nach einem der Ansprüche 5 und 7, **dadurch gekennzeichnet, dass** die Befestigungsmittel (15) nach Art von Rasthaken ausgebildet sind.

10. Fluidventil- und -verbindungsvorrichtung (1) nach einem der vorhergehenden Ansprüche, **dadurch gekennzeichnet, dass** das erste und/oder das zweite externe Anschlusselement (5, 20) nach Art einer Hohlkanüle ausgebildet ist.

11. Fluidventil- und -verbindungsvorrichtung (1) nach einem der vorhergehenden Ansprüche, **dadurch gekennzeichnet, dass** das zweite externe Anschlusselement (31) mit einem oder mit zwei Kanälen (32, 33) ausgebildet ist.

12. Fluidventil- und -verbindungsvorrichtung (1) nach einem der vorhergehenden Ansprüche, **dadurch gekennzeichnet, dass** das erste interne Anschlusselement (34) ein hohlzylindrischer Stutzen ist und das zweite interne Anschlusselement (35) ein hohlzylindrischer Stutzen ist, welcher bei Verlagerung zwischen der ersten Position und der zweiten Position ineinander greifen.

13. Fluidventil- und -verbindungsvorrichtung (1) nach einem der vorhergehenden Ansprüche, **dadurch gekennzeichnet, dass** das zweite interne Anschlusselement (35) ein hohlzylindrischer Stutzen ist, welcher bei Verlagerung zwischen der ersten Position und der zweiten Position das Ventilelement (6) beaufschlagt.

14. Fluidventil- und -verbindungsvorrichtung (1) nach einem der vorhergehenden Ansprüche, **dadurch gekennzeichnet, dass** das Ventilelement (6) in der ersten Position des Betätigungselements (16) die Fluidverbindung zwischen dem ersten und dem zweiten externen Anschlusselement (5, 20, 31, 38) unterbindet und das Ventilelement (6) in der zweiten Position des Betätigungselements (16) die Fluidverbindung zwischen dem ersten und dem zweiten externen Anschlusselement (5, 20, 31, 38) ermöglicht.

15. Fluidventil- und -verbindungsvorrichtung (1) nach einem der vorhergehenden Ansprüche, **dadurch gekennzeichnet, dass** das Ventilelement (6) einen etwa zylindrischen Bereich mit von dem zylindrischen Bereich abragende Federarme aufweist.

16. Fluidventil- und -verbindungsvorrichtung (1) nach einem der vorhergehenden Ansprüche, **dadurch gekennzeichnet, dass** das Ventilelement (6) mit seinem etwa zylindrischen Bereich in dem ersten internen Anschlusselement (4, 34) angeordnet ist, wobei sich die Federarme jeweils an einer Innenwandung des Gehäuses (2) abstützen.

## Claims

1. A fluid valve and fluid connection system (1) with a housing (2) with a receiving chamber (3) with a first internal connection element (4) and with a first external connection element (5), with a first movable valve element (6) arranged in the receiving chamber (3) and with an actuating element (16) which has a fluid communication system with a second internal connection element (19) and with a second external connection element (20), wherein the first internal connection element (4) cooperates with the second internal connection element (19) and the valve element (6) in order to establish or to block a fluid connection between the first and the second external connection element (5, 20), wherein the receiving chamber (3) is arranged in the housing (2),
wherein the first internal connection element (4) and the first external connection element (5) protrude from a respective wall of the housing (2) in an axial direction,
wherein the first internal connection element (4) and the first external connection element (5) can be connected to one another via the receiving chamber (3),
wherein by shifting the actuating element (16) the valve element (6) is actuated in order to establish a fluid connection between the first external connection element (5) and the second external connection element (20),
wherein the actuating element (16) can be moved relative to the housing (2) and can adopt a first position, in which the fluid connection between the first and the second external connection element (5, 20) is blocked, and can adopt a second position, in which the fluid connection between the first and the second external connection element (5, 20) is established,
wherein the actuating element (16) has means (21, 22) that cooperate with means (23) of the housing (2) in order to define the first and the second position, wherein the means of the actuating element (16) have at least one arm (21), which has at least one radially inwardly oriented protrusion (22), and wherein the means of the housing (2) are grooves (23) formed in a housing wall,
and a blocking element (24) is provided, which when in a first position prevents the actuating element (16) from adopting the second position and which when in a second position allows the actuating element (16) to adopt the second position.

2. The fluid valve and fluid connection system (1) as claimed in claim 1, **characterised in that** the grooves (23) are arranged spaced apart from one another in the axial direction of the housing (2).

3. The fluid valve and fluid connection system (1) as claimed in claim 1 or 2, **characterised in that** the at least one arm is arranged in the first position in such a way that the at least one protrusion (22) engages with one of the grooves (23) and the at least one arm (21) is arranged in the second position in such a way that the at least one protrusion (22) engages with another one of the grooves (23).

4. The fluid valve and fluid connection system (1) as claimed in any one of the preceding claims, **characterised in that** the blocking element (24) is an open ring element, which grasps around the housing (2) and prevents the actuating element (16) from adopting the second position **in that** the position of the means (23) of the actuating element (16) is limited in such a way that the at least one protrusion (22) cannot engage with the groove (23) associated with the second position.

5. The fluid valve and fluid connection system (1) as claimed in any one of the preceding claims, **characterised in that** the housing (2) is provided with fastening means (15) in order to fasten the housing (2) to a second storage vessel.

6. The fluid valve and fluid connection system (1) as claimed in any one of the preceding claims, **characterised in that** the housing (2) is provided with the first external connection element (5) in order to establish a fluid connection to the second storage vessel.

7. The fluid valve and fluid connection system (1) as claimed in any one of the preceding claims, **characterised in that** the actuating element (16) is provided with fastening means (15) in order to fasten the housing (2) to a first storage vessel.

8. The fluid valve and fluid connection system (1) as claimed in any one of the preceding claims, **characterised in that** the actuating element (16) is provided with the second external connection element (20) in order to establish a fluid connection to the first storage vessel.

9. The fluid valve and fluid connection system (1) as claimed in any one of claims 5 and 7, **characterised in that** the fastening means (15) are formed in the manner of detent arms.

10. The fluid valve and fluid connection system (1) as claimed in any one of the preceding claims, **characterised in that** the first and/or the second external connection element (5, 20) is/are formed in the manner of a hollow cannula.

11. The fluid valve and fluid connection system (1) as claimed in any one of the preceding claims, **characterised in that** the second external connection element (31) is formed with one or with two channels (32, 33).

12. The fluid valve and fluid connection system (1) as claimed in any one of the preceding claims, **characterised in that** the first internal connection element (34) is a hollow-cylindrical connecting piece and the second internal connection element (35) is a hollow-cylindrical connecting piece, which engage with one another when moved between the first position and the second position.

13. The fluid valve and fluid connection system (1) as claimed in any one of the preceding claims, **characterised in that** the second internal connection element (35) is a hollow-cylindrical connecting piece which, when moved between the first position and the second position, acts on the valve element (6).

14. The fluid valve and fluid connection system (1) as claimed in any one of the preceding claims, **characterised in that** the valve element (6) in the first position of the actuating element (16) blocks the fluid connection between the first and the second external connection element (5, 20, 31, 38) and the valve element (6) in the second position of the actuating element (16) allows the fluid connection between the first and the second external connection element (5, 20, 31, 38).

15. The fluid valve and fluid connection system (1) as claimed in any one of the preceding claims, **characterised in that** the valve element (6) has an approximately cylindrical region with spring arms protruding away from the cylindrical region.

16. The fluid valve and fluid connection system (1) as claimed in any one of the preceding claims, **characterised in that** the valve element (6) is arranged with its approximately cylindrical region in the first internal connection element (4, 34), wherein the spring arms are each supported on an inner wall of the housing (2).

## Revendications

1. Dispositif de soupape et de communication fluidique (1), ledit dispositif comprenant un logement (2) comportant un espace de réception (3) ayant un premier élément de raccordement interne (4) et ayant un premier élément de raccordement externe (5), ledit dispositif comprenant un premier élément de soupape mobile (6) disposé dans l'espace de réception (3) et comprenant un élément d'actionnement (16) qui présente un dispositif de communication fluidique comportant un second élément de raccordement interne (19) et un second élément de raccordement externe (20), où le premier élément de raccordement interne (4) et le second élément de raccordement interne (19), qui lui est associé, fonctionnent de façon conjointe avec l'élément de soupape (6), afin de réaliser ou d'empêcher une communication fluidique entre le premier et le second élément de raccordement externe (5, 20), où l'espace de réception (3) est disposé dans le logement (2),
le premier élément de raccordement interne (4) et le premier élément de raccordement externe (5) sont à chaque fois en saillie, dans une direction axiale, par rapport à une paroi du logement (2),
le premier élément de raccordement interne (4) et le premier élément de raccordement externe (5) peuvent être reliés l'un à l'autre par l'espace de réception (3),
où l'élément de soupape (6) est actionné par le déplacement de l'élément d'actionnement (16), afin de réaliser une communication fluidique entre le premier élément de raccordement externe (5) et le second élément de raccordement externe (20),
où l'élément d'actionnement (16) peut être déplacé par rapport au logement (2) et peut occuper une première position dans laquelle est empêchée la communication fluidique entre le premier et le second élément de raccordement externe (5, 20), et ledit élément d'actionnement (16) peut occuper une seconde position dans laquelle est réalisée la communication fluidique se produisant entre le premier et le second élément de raccordement externe (5, 20),
où l'élément d'actionnement (16) présente des moyens (21, 22) qui agissent de façon conjointe avec des moyens (23) du logement (2), afin de définir la première et la seconde position,
où les moyens de l'élément d'actionnement (16) présentent au moins un bras (21) qui présente au moins une partie saillante (22) orientée vers l'intérieur dans le sens radial, et où les moyens du logement (2) sont des rainures (23) formées dans une paroi du logement,
et il est prévu un élément de blocage (24) qui, en se trouvant dans une première position, empêche l'élément d'actionnement (16) d'occuper la seconde position et qui, en se trouvant dans une seconde position, permet à l'élément d'actionnement (16) d'occuper la seconde position.

2. Dispositif de soupape et de communication fluidique (1) selon la revendication 1, **caractérisé en ce que** les rainures (23) sont disposées en étant espacées les unes des autres suivant la direction axiale du logement (2).

3. Dispositif de soupape et de communication fluidique (1) selon la revendication 1 ou 2, **caractérisé en ce que** le bras au moins au nombre de un est disposé dans la première position de manière telle, que la partie saillante (22) au moins au nombre de un s'engage dans l'une des rainures (23), et le bras (21) au moins au nombre de un est disposé dans la seconde position de manière telle, que la partie saillante (22) au moins au nombre de un s'engage dans une autre des rainures (23) formées.

4. Dispositif de soupape et de communication fluidique (1) selon l'une quelconque des revendications précédentes, **caractérisé en ce que** l'élément de blocage (24) est un élément annulaire ouvert qui enserre le logement (2) et qui empêche l'élément d'actionnement (16) d'occuper la seconde position, par le fait que la position des moyens (23) de l'élément d'actionnement (16) est limitée de manière telle, que la partie saillante (22) au moins au nombre de un ne puisse pas s'engager dans la rainure (23) associée à la seconde position.

5. Dispositif de soupape et de communication fluidique (1) selon l'une quelconque des revendications précédentes, **caractérisé en ce que** le logement (2) est doté de moyens de fixation (15), afin de fixer le logement (2) sur un second récipient formant réservoir.

6. Dispositif de soupape et de communication fluidique (1) selon l'une quelconque des revendications précédentes, **caractérisé en ce que** le logement (2) est doté du premier élément de raccordement externe (5), afin de réaliser une communication fluidique avec le second récipient formant réservoir.

7. Dispositif de soupape et de communication fluidique (1) selon l'une quelconque des revendications précédentes, **caractérisé en ce que** l'élément d'actionnement (16) est doté de moyens de fixation (15), afin de fixer le logement (2) sur un premier récipient formant réservoir.

8. Dispositif de soupape et de communication fluidique (1) selon l'une quelconque des revendications précédentes, **caractérisé en ce que** l'élément d'actionnement (16) est doté du second élément de raccordement externe (20), afin de réaliser une communication fluidique avec le premier récipient formant réservoir.

9. Dispositif de soupape et de communication fluidique (1) selon l'une des revendications 5 et 7, **caractérisé en ce que** les moyens de fixation (15) sont conçus à la manière de crochets d'encliquetage.

10. Dispositif de soupape et de communication fluidique (1) selon l'une quelconque des revendications précédentes, **caractérisé en ce que** le premier et / ou le second élément de raccordement externe (5, 20) est conçu à la manière d'une canule creuse.

11. Dispositif de soupape et de communication fluidique (1) selon l'une quelconque des revendications précédentes, **caractérisé en ce que** le second élément de raccordement externe (31) est conçu en ayant un ou deux conduits (32, 33).

12. Dispositif de soupape et de communication fluidique (1) selon l'une quelconque des revendications précédentes, **caractérisé en ce que** le premier élément de raccordement interne (34) est une tubulure cylindrique creuse et le second élément de raccordement interne (35) est une tubulure cylindrique creuse, tubulures qui, en passant de la première position à la seconde position, s'engagent l'une dans l'autre.

13. Dispositif de soupape et de communication fluidique (1) selon l'une quelconque des revendications précédentes, **caractérisé en ce que** le second élément de raccordement interne (35) est une tubulure cylindrique creuse qui, en passant de la première position à la seconde position, sollicite l'élément de soupape (6).

14. Dispositif de soupape et de communication fluidique (1) selon l'une quelconque des revendications précédentes, **caractérisé en ce que** l'élément de soupape (6), quand l'élément d'actionnement (16) se trouve dans la première position, empêche la communication fluidique entre le premier et le second élément de raccordement externe (5, 20, 31, 38), et l'élément de soupape (6), quand l'élément d'actionnement (16) se trouve dans la seconde position, permet la communication fluidique entre le premier et le second élément de raccordement externe (5, 20, 31, 38).

15. Dispositif de soupape et de communication fluidique (1) selon l'une quelconque des revendications précédentes, **caractérisé en ce que** l'élément de soupape (6) présente une zone sensiblement cylindrique comprenant des bras élastiques faisant saillie par rapport à la zone cylindrique.

16. Dispositif de soupape et de communication fluidique (1) selon l'une quelconque des revendications précédentes, **caractérisé en ce que** l'élément de soupape (6), avec sa zone sensiblement cylindrique, est disposé dans le premier élément de raccordement interne (4, 34), où les bras élastiques s'appuient à chaque fois sur une paroi intérieure du logement (2).
